# EUROPEAN PATENT APPLICATION

(11) **EP 4 640 207 A1**
(43) Date of publication of application: **29.10.2025**
(21) Application number: 23907096.4
(22) Date of filing: 20.12.2023
(51) Int. Cl.: A61K 8/67, A61K 8/34, A61K 9/08, A61K 31/7048, A61K 47/10, A61P 17/00, A61Q 19/00

(54) **AQUEOUS COMPOSITION AND EXTERNAL PREPARATION FOR SKIN**

(30) Priority: 23.12.2022 JP 2022206769
(71) Applicant: Resonac Corporation, Tokyo 105-7325 (JP)
(72) Inventor: NAKAGAMI, Yuko, Tokyo 105-7325 (JP); OHTAKE, Noriko, Tokyo 105-7325 (JP)
(74) Representative: Strehl & Partner mbB
(86) International application number: PCT/JP2023/045728
(87) International publication number: WO 2024/135736

(57) **Abstract**

An aqueous composition contains a glycoside of vitamin E, represented by General Formula (1), 1,2-hexanediol, and water. In Formula (1), R¹, R², and R³ each independently represents a hydrogen atom or a methyl group, Rx represents a monosaccharide, and Ry represents a group represented by Formula (Ry-1) or (Ry-2). * represents a bonding site to a carbon atom to which Ry in General Formula (1) is bonded. An external preparation for skin, containing the aqueous composition, is also provided.

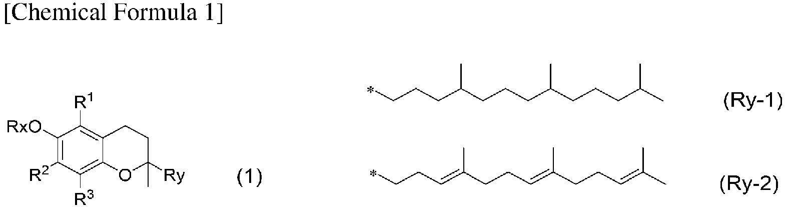

## Description

### TECHNICAL FIELD

The present invention relates to an aqueous composition and an external preparation for skin.

Priority is claimed on Japanese Patent Application No. 2022-206769, filed on December 23, 2022, the content of which is incorporated herein by reference.

### BACKGROUND ART

Patent Document 1 discloses an anti-allergic agent containing tocopheryl glycoside as an active ingredient. Examples of the tocopheryl glycoside include tocopheryl glucoside.

Patent Document 2 discloses a delayed-type hypersensitivity prevention and treatment agent, an immunoglobulin E antibody production inhibitor, and an anti-inflammatory agent, each of which contains tocopheryl glucoside as an active ingredient.

### Citation List

### Patent Documents

Patent Document 1: Japanese Unexamined Patent Application, First Publication No. S61-030594
Patent Document 2: PCT International Publication No. WO1991/007179

### SUMMARY OF INVENTION

### Technical Problem

Since a glycoside of vitamin E (tocopherol or tocotrienol) has significantly low solubility in water, it is difficult to prepare the glycoside of vitamin E as an aqueous composition.

Therefore, an object of the present invention is to provide an aqueous composition of a glycoside of vitamin E, and an external preparation for skin, containing the aqueous composition.

### Solution to Problem

The present invention includes the following aspects.
[1] An aqueous composition containing:
   a glycoside of vitamin E, represented by General Formula (1);
   1,2-hexanediol; and
   water, 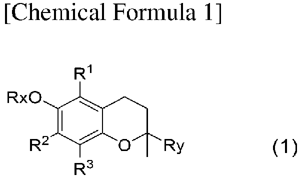
   [in the formula, R¹, R², and R³ each independently represents a hydrogen atom or a methyl group, Rx represents a monosaccharide, and Ry represents a group represented by Formula (Ry-1) or (Ry-2)] 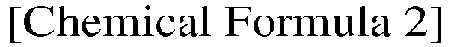 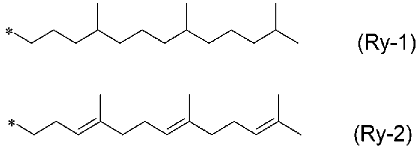 [in the formula, * represents a bonding site to a carbon atom to which Ry in General Formula (1) is bonded]
[2] The aqueous composition according to [1],
   in which the aqueous composition contains 0.01% to 2% by mass of the glycoside of vitamin E and 0.5% to 20% by mass of the 1,2-hexanediol.
[3] The aqueous composition according to [1] or [2],
   in which the glycoside of vitamin E is a glucoside of vitamin E.
[4] The aqueous composition according to [1] or [2],
   wherein the glycoside of vitamin E is tocopheryl glucoside.
[5] An external preparation for skin, containing:
   the aqueous composition according to any one of [1] to [4].

### Advantageous Effects of Invention

According to the present invention, an aqueous composition of a glycoside of vitamin E, and an external preparation for skin, containing the aqueous composition are provided.

### DESCRIPTION OF EMBODIMENTS

Hereinafter, embodiments according to the present invention will be described in detail. However, the present invention is not limited to the embodiments described below.

### (Aqueous composition)

In one embodiment, the present invention provides an aqueous composition. The aqueous composition according to the present embodiment contains a glycoside of vitamin E, represented by General Formula (1), 1,2-hexanediol, and water. 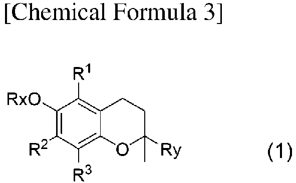 [in the formula, R¹, R², and R³ each independently represents a hydrogen atom or a methyl group, Rx represents a monosaccharide, and Ry represents a group represented by Formula (Ry-1) or (Ry-2)] 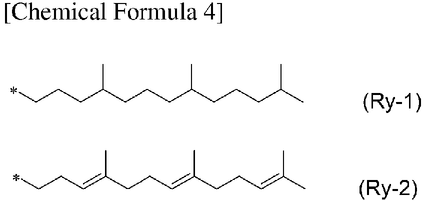 [in the formula, * represents a bonding site to a carbon atom to which Ry in General Formula (1) is bonded]

### <Glycoside of vitamin E>

In the present specification, a compound in which a monosaccharide is bonded to vitamin E is referred to as a glycoside of vitamin E. The vitamin E is tocopherol and tocotrienol; and may be tocopherol alone, tocotrienol alone, or a mixture of both compounds. More specifically, the glycoside of vitamin E is a compound in which a monosaccharide is bonded to tocopherol or tocotrienol. More specifically, the glycoside of vitamin E is a compound in which tocopherol or tocotrienol is bonded to a carbon atom (asymmetric carbon atom) at an anomeric position of a monosaccharide. When Ry in General Formula (1) is a group represented by Formula (Ry-1), the glycoside of vitamin E is a compound (tocopheryl glycoside) in which tocopherol is bonded to a carbon atom at an anomeric position of a monosaccharide. When Ry in General Formula (1) is a group represented by Formula (Ry-2), the glycoside of vitamin E is a compound (tocotrienyl glycoside) in which tocotrienol is bonded to a carbon atom at an anomeric position of a monosaccharide. The glycoside of vitamin E may be a mixture of a compound in which tocopherol is bonded to a carbon atom at an anomeric position of a monosaccharide and a compound in which tocotrienol is bonded to a carbon atom at an anomeric position of a monosaccharide.

Tocotrienol may be chemically unstable. On the other hand, tocopherol is easily available as a chemically synthesized product, and is more chemically stable. Therefore, when used for applications such as an external preparation for skin, it is preferable that the glycoside of vitamin E contain tocopheryl glycoside as a main component or consists of tocopheryl glycoside.

Tocopherol is a compound represented by General Formula (2). Tocotrienol is a compound represented by General Formula (3). 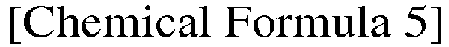 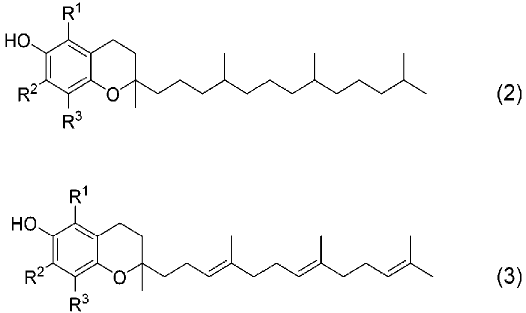 [in the formula, R¹, R², and R³ each independently represents a hydrogen atom or a methyl group]

In tocopherol and tocotrienol, α-tocopherol and α-tocotrienol (R¹, R², R³ = CH₃), β-tocopherol and β-tocotrienol (R¹, R³ = CH₃, and R² = H), γ-tocopherol and γ-tocotrienol (R¹ = H, and R², R³ = CH₃), and δ-tocopherol and δ-tocotrienol (R¹, R² = H, and R³ = CH₃) are present depending on R¹, R², and R³ in General Formulae (2) and (3).

Tocopherol may be any of the α-tocopherol, the β-tocopherol, the γ-tocopherol, or the δ-tocopherol. Tocotrienol may be any of the α-tocotrienol, the β-tocotrienol, the γ-tocotrienol, or the δ-tocotrienol.

Since both tocopherol and tocotrienol have an asymmetric carbon atom at the 2-position of a chromane ring, stereoisomers of a d-form, an l-form and a dl-form are present. Either of tocopherol and tocotrienol may be any of these stereoisomers thereof. Although both tocopherol and tocotrienol have characteristic physiological activity, since they have similar chemical properties, a mixture of tocopherol and tocotrienol may be treated as the vitamin E, or products sold as tocopherol may contain a small amount of tocotrienol. In addition, products sold as tocotrienol may contain a small amount of tocopherol.

Rx in General Formula (1) represents a monosaccharide. More specifically, Rx is a monovalent group obtained by removing a hydroxyl group bonded to a carbon atom at an anomeric position from the monosaccharide. Examples of the monosaccharide include glucose, mannose, galactose, fucose, rhamnose, and arabinose. The monosaccharide may be the D-form, the L-form, or a mixture of these forms. The monosaccharide may be an α-form or a β-form. As the monosaccharide, glucose is preferable. A compound in which vitamin E and glucose are bonded to each other is referred to as a glucoside of vitamin E. In addition, a compound in which tocopherol and glucose are bonded to each other is referred to as tocopheryl glucoside. A compound in which tocotrienol and glucose are bonded to each other is referred to as tocotrienyl glucoside.

A specific example of the glucoside of vitamin E is a compound in which δ-tocopherol and β-D-glucose are bonded at an anomeric position, which is represented by Formula (4). A compound in which δ-tocotrienol and β-D-glucose are bonded at an anomeric position is represented by Formula (5).

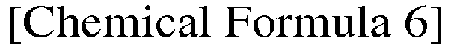

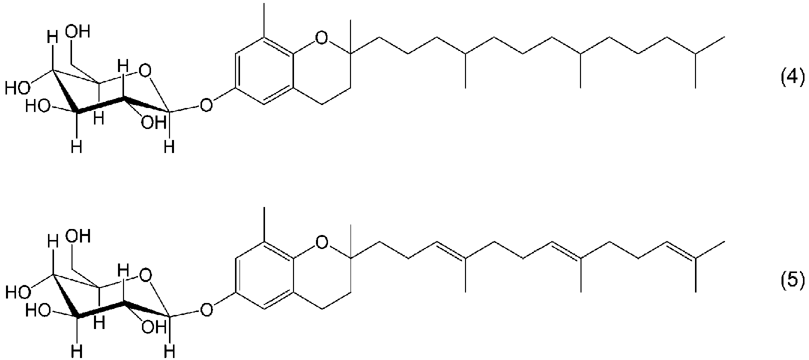

The glycoside of vitamin E can be synthesized by reacting tocopherol or tocotrienol with a glycoside derivative by a known reaction. For example, as in the method described in Japanese Unexamined Patent Application, First Publication 2008-133275, tocopherol or tocotrienol is reacted with a hyperacetylated sugar in the presence of an appropriate acid catalyst. Next, a derivative in which tocopherol or tocotrienol is bonded to an anomeric position of the hyperacetylated sugar is obtained. Next, the glycoside of vitamin E can be synthesized by removing an acetyl group from the derivative by a deprotection reaction of the acetyl group. When a material used as the vitamin E is a mixture of tocopherol and tocotrienol, a mixture of compounds having structures derived from tocopherol and tocotrienol is obtained as the glycoside of vitamin E.

### <1,2-Hexanediol>

As the 1,2-hexanediol, a grade which can be used as a cosmetic component can be used. For example, a product commercially available from KANKOHSHA Co.,Ltd. under the trade name KMO-6 can be used.

### <Water>

As the water, a grade which can be used in cosmetics, such as purified water, can be used.

### <Aqueous composition>

The term "aqueous composition" refers to a composition in which a solute is dissolved in a solvent mainly composed of water. In the aqueous composition according to the present embodiment, at least the glycoside of vitamin E is dissolved as a solute in a mixed solvent containing water and 1,2-hexanediol. In the aqueous composition, a proportion of the water in the mixed solvent is preferably 60% by mass or more, more preferably 70% by mass or more, still more preferably 80% by mass or more, and particularly preferably 90% by mass or more with respect to the total mass of the mixed solvent.

A content of water in the aqueous composition is preferably 80% by mass or more, more preferably 90% by mass or more, and still more preferably 95% by mass or more with respect to the total mass of the aqueous composition. The content of water in the aqueous composition is preferably 80% to 99.4% by mass, more preferably 90% to 99% by mass, and still more preferably 95% to 98% by mass with respect to the total mass of the aqueous composition.

A content of the glycoside of vitamin E in the aqueous composition according to the present embodiment is preferably 0.01% to 2% by mass with respect to the total mass of the aqueous composition. The content of the glycoside of vitamin E is more preferably 0.1% to 1.0% by mass, and still more preferably 0.2% to 1.0% by mass.

A content of 1,2-hexanediol in the aqueous composition according to the present embodiment is preferably 0.5% to 20% by mass with respect to the total mass of the aqueous composition. The content of 1,2-hexanediol is more preferably 1% to 10% by mass, and still more preferably 2% to 5% by mass.

In the aqueous composition according to the present embodiment, when dissolving the glycoside of vitamin E using the effect of 1,2-hexanediol, an organic solvent other than 1,2-hexanediol may be contained as the solvent. The above-described organic solvent can be appropriately selected according to the use application of the aqueous composition. For example, when the above-described organic solvent is a water-soluble alcohol such as ethanol, it is preferable that a content of the water-soluble alcohol be kept at 5% by mass or less (that is, 0% to 5% by mass) with respect to the total mass of the aqueous composition, to the extent that the effect of 1,2-hexanediol is not impaired.

When the contents of the glycoside of vitamin E and 1,2-hexanediol in the aqueous composition are within the above-described preferred ranges, the glycoside of vitamin E is sufficiently dissolved, and as a result, the physiological activity of the glycoside of vitamin E can be sufficiently exhibited.

In the aqueous composition, when the amount of 1,2-hexanediol is 20% by mass or less, the risk of unwanted effects such as irritancy and the like at the application site is further reduced as the aqueous composition is applied as an external preparation for skin. Therefore, the content of 1,2-hexanediol in the aqueous composition is preferably 20% by mass or less.

The aqueous composition according to the present embodiment can be produced by dissolving the glycoside of vitamin E in a mixed solvent of water and 1,2-hexanediol. For example, the mixed solvent of water and 1,2-hexanediol is put into an appropriate container, the glycoside of vitamin E is further added thereto, and the mixture is stirred to dissolve the glycoside of vitamin E, thereby obtaining an aqueous composition. The aqueous composition according to the present embodiment is generally a colorless and transparent liquid material.

In regard to the aqueous composition according to the present embodiment, the present inventors have assesed the effect of promoting the expression of a transient receptor potential vanilloid 4 (TRPV4) gene using an aqueous composition containing tocopheryl glucoside as an example, and found that the aqueous composition has excellent effects of promoting the expression of TRPV4.

TRPV4 is a protein which has been found to be an osmotically sensitive receptor activated at a low osmotic pressure. TRPV4 has a function as a moisture evaporation sensor, and it has been reported that TRPV4 suppresses moisture evaporation and enhances a skin barrier function by activation with 4α-phorbol ester (4α-PDD).

A tight junction (TJ) is deeply involved in the skin barrier function. The TJ is an intercellular adhesion structure which is formed in a belt shape around epithelial cells. The TJ closely attaches adjacent epithelial cells to each other and closes a gap between the cells, thereby preventing a substance from entering and exiting through the gap between the cells. The TJ is developed in the skin, the trachea, the intestinal tract, the blood vessel, and the like, and has a barrier function in which a liquid inside the tissue does not permeate and foreign substances from the outside do not enter. Claudin (CLDN), occludin (OCL), and the like are known as membrane proteins localized in the TJ. It has been known that the claudin has 24 members, and among them, claudin-1 (CLDN1) is responsible for strand formation at the TJ and plays an important role in the formation of the intercellular barrier in the epidermis of the skin.

It has been reported that the activation of the TRPV4 present in the epidermis promotes the formation of the TJ, improves the skin barrier function, and thus is effective for improvement of rough skin and moisturizing of the skin.

The aqueous composition according to the present embodiment has an action of promoting the expression of the TRPV4 gene, the CLDN1 gene, and the OCL gene, and increasing the amounts of TRPV4, OLDN1, and OCL. In addition, the aqueous composition according to the present embodiment has an action of suppressing transepidermal water loss. Therefore, the aqueous composition according to the present embodiment can be used as an excellent skin roughness improving agent and/or a skin moisturizer.

### (External preparation for skin)

In one embodiment, the present invention provides an external preparation for skin. The external preparation for skin according to the present embodiment contains the above-described aqueous composition.

When the aqueous composition according to the present embodiment is used as an external preparation for skin, other components may be contained in addition to the aqueous composition. Examples of the other components include a pharmacologically acceptable carrier.

In the present specification, the term "pharmacologically acceptable carrier" means a carrier which does not inhibit the physiological activity of an active ingredient and does not exhibit substantial toxicity with respect to the administration target.

The term "does not exhibit substantial toxicity" means that this component does not exhibit toxicity with respect to an administration target in a generally used dosage.

The pharmacologically acceptable carrier is not particularly limited; and examples thereof include excipients, binders, disintegrants, lubricants, stabilizers, diluents, solvents for injections, moisturizers, touch sensation improvers, surfactants, polymer-thickening-gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, wetting agents, thickeners, tackifiers, oily raw materials, liquid matrices, fat-soluble substances, and polymer carboxylate salts.

For the pharmacologically acceptable carrier in the barrier-function-improving composition according to the present embodiment, one kind may be used alone, or two or more kinds may be used together.

In addition, examples of the other components include preservatives, antibacterial agents, ultraviolet absorbents, whitening agents, vitamins and derivatives thereof, antiphlogistics, anti-inflammatory agents, hair growth drugs, blood circulation promoters, stimulants, hormones, anti-wrinkle agents, anti-aging agents, tightening agents, cooling sensation agents, warming sensation agents, wound healing promoters, irritation relieving agents, analgesics, cell-activating agents, plant extracts, animal extracts, microbial extracts, seed oils, antipruritic agents, keratin exfoliating and dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, anti-itch drugs, keratin softening and exfoliating agents, ultraviolet blockers, disinfectants, antioxidant substances, pH adjusters, additives, and metal soaps. Specific examples of these components include components described in PCT International Publication No. WO2016/076310. Among these components, one kind may be used alone, or two or more kinds may be used together.

The external preparation for skin according to the present embodiment can be produced by formulating the above-described aqueous composition and optionally the pharmacologically acceptable carrier and other components by mixing them together according to a conventional method (for example, a method described in the Japanese Pharmacopoeia).

The external preparation for skin according to the present embodiment may be a cosmetic. Examples of the type of the cosmetic material include hair cosmetic materials such as shampoo, conditioning shampoo, dandruff shampoo, hair color shampoo, rinse-integrated shampoo, rinse, treatment, hair foam, hair mousse, hair spray, hair mist, hair gel, water grease, set lotion, color lotion, hair tonic, hair liquid, hair blow, split-end coating, permanent wave agent, straight perm agent, oxidative hair dyeing agent, hair bleaching agent, hair color pretreatment, hair color aftertreatment, perm pretreatment, perm aftertreatment, hair manicure, and hair growth agent; basic cosmetics such as facial wash, cleansing foam, washing powder, facial washing powder, cleansing milk, cleansing lotion, cleansing gel, cleansing mask, lotion, softening lotion, astringent lotion, facial washing lotion, multi-layered lotion, emulsion, emollient lotion, moisturizing lotion, milky lotion, nourishing lotion, nourishing milk, skin moisture, moisture emulsion, massage lotion, cleansing lotion, protect emulsion, sun protect, sun protector, UV care milk, sunscreen, makeup lotion, rough skin smoother, elbow lotion, hand lotion, body lotion, cream, emollient cream, nutritional cream, vanishing cream, moisture cream, night cream, massage cream, cleansing cream, makeup cream, base cream, pre-makeup cream, sunscreen cream, sun tank cream, hair removal cream, deodorant cream, shaving cream, rough skin softening cream, gel, cleansing gel, moisture gel, soap, facial soap, transparent soap, medicinal soap, liquid soap, shaving soap, synthetic facial soap, pack, masks, peel-off packs, powder packs, washing packs, oil packs, cleansing masks, essence, moisturizing essence, whitening essence, ultraviolet ray-preventing essence, liposome beauty liquid, and liposome skin lotion; makeup cosmetics such as white face powder or dusting powder substances, foundation, makeup base, cheek rouge, eye liner, mascara, eye shadow, eyebrow ink, eyebrow, nail enamel, enamel remover, and nail treatment; aromatic cosmetics such as perfume, parfum, eau de parfum, eau de toilette, eau de cologne, perfume concentrate, scented powder, perfumed soap, body lotion, and bath oil; body cosmetics such as body shampoo, body washing agent, body powder, deodorant lotion, deodorant powder, deodorant spray, deodorant stick, deodorant agent, bleaching agent, hair removal and depilatory agent, bath agent, insect repellent spray, and insect repellent agent; and ointment, plaster, lotion, liniment, and liquid coating agent.

### (Other embodiments)

In one embodiment, the present invention provides a method for moisturizing skin, a method for improving dry skin, or a method for improving rough skin, each of which includes a step of applying the above-described aqueous composition to target skin.

In one embodiment, the present invention provides a method for promoting the production of TRPV4, CLDN1, and OCL1, the method including a step of applying the above-described aqueous composition to target skin.

In one embodiment, the present invention provides the above-described aqueous composition for moisturizing skin, improving dry skin, or improving rough skin.

In one embodiment, the present invention provides the above-described aqueous composition for promoting the production of TRPV4, CLDN1, and OCL1 .

In one embodiment, the present invention provides the use of the above-described aqueous composition for producing an external preparation for skin, for improving skin moisturizing, improving dry skin, or improving rough skin.

In one embodiment, the present invention provides the use of the above-described aqueous composition for producing a production promoter of TRPV4, CLDN1, and OCL1.

In one embodiment, the present invention provides the use of the above-described aqueous composition for moisturizing the skin, improving dry skin, or improving skin roughness.

In one embodiment, the present invention provides the use of the above-described aqueous composition for promoting the production of TRPV4, CLDN1, and OCL1.

### Example

Hereinafter, the present invention will be described in more detail with reference to Examples and Comparative Examples, but the present invention is not limited to the following Examples.

D-δ-Tocopheryl glucoside used in the following experiments was synthesized with reference to the synthesis method described in Japanese Unexamined Patent Application, First Publication 2008-133275 and the like.

### [Experimental Example 1]

### (Confirmation of solubility of tocopheryl glucoside)

The solubility of the D-δ-tocopheryl glucoside (hereinafter, may be abbreviated as "δ-TPG") was examined.

As shown in Tables 1 and 2, water and 1,2-hexanediol (hereinafter, may be abbreviated as "HD") or ethanol (hereinafter, may be abbreviated as "EtOH") were mixed to prepare a mixture of each example. 9.9 g of each of the mixtures was placed in individual vials, 0.01 g of solid δ-TPG was added to each vial, and the mixture was stirred at room temperature. After stirring for 1 hour, the state of each vial was observed. Based on the following evaluation standard, the solubility of δ-TPG was evaluated from the observation results of the vials. The results are shown in Table 1 and Table 2 as "Solubility evaluation".

### <Evaluation standard>

A: the solution was completely dissolved and clear.
B: precipitation or turbidity occurred.

Table 1 shows the results (Examples 1 to 4) of dissolving 0.01 g of δ-TPG in each of the mixtures of water and HD. Table 2 shows the results (Comparative Examples 1 and 2) of dissolving 0.01 g of δ-TPG in each of the mixtures of water and EtOH.

**[Table 1]**

| | Formulation mixture | | Content of HD in aqueous composition when δ-TPG (0.01 g) is dissolved | Evaluation of solubility |
|---|---|---|---|---|
| | Water (g) | HD (g) | | |
| Example 1 | 9.94 | 0.05 | 0.5 | A |
| Example 2 | 9.59 | 0.5 | 5 | A |
| Example 3 | 8.99 | 1.0 | 10 | A |
| Example 4 | 7.49 | 2.5 | 25 | A |

**[Table 2]**

| | Formulation mixture | | Content of EtOH in aqueous composition when δ-TPG (0.01 g) is dissolved | Evaluation of solubility |
|---|---|---|---|---|
| | Water (g) | EtOH (g) | | |
| Comparative Example 1 | 9.94 | 0.5 | 5 | B |
| Comparative Example 2 | 8.99 | 1.0 | 10 | B |

δ-TPG did not dissolve in water. However, δ-TPG completely dissolved in EtOH or HD. As the aqueous composition, it is not preferable to use EtOH or HD at a high concentration in the aqueous composition. Therefore, the solubility of δ-TPG when these compounds were reduced, that is, the solubility of δ-TPG in the aqueous composition containing EtOH or HD at a lower concentration was examined. When the mixture contained 5% by mass or 10% by mass of EtOH, δ-TPG did not dissolve (Table 2). On the other hand, when the mixture was prepared using HD, δ-TPG dissolved even in a mixture having a low HD concentration of approximately 0.5% by mass, and thus a clear aqueous composition could be obtained (Table 1).

### [Experimental Example 2]

(Confirmation of activity of aqueous composition containing δ-TPG and HD: expression of TRPV4 gene)
Normal human epidermal keratinocytes (NHEK, manufactured by KURABO INDUSTRIES LTD.) were seeded in a plastic petri dish at a seeding density of 10,000 cells/cm², and cultured for 24 hours in a normal human epidermal keratinocyte serum-free culture medium (manufactured by KURABO INDUSTRIES LTD.).

8.94 g of water, 1.0 g of HD, and 0.06 g of δ-TPG were mixed and stirred to obtain an aqueous composition. The above-described aqueous composition was added to a culture solution in which the above-described NHEK cells had been cultured for 24 hours, such that the final concentration of δ-TPG was 10 µM.

After adding the δ-TPG, the above-described culture solution was further cultured for 48 hours. Thereafter, the culture solution was irradiated with UVB at 60 mJ/cm² using a UVB irradiation device of UVP, LLC. Thereafter, a fresh normal human epidermal keratinocyte growth medium was added to the above-described culture solution, and the culture was further carried out for 24 hours. In this manner, evaluation cells were obtained.

Total RNA was extracted from the evaluation cells obtained above, and cDNA was synthesized. Subsequently, quantitative real-time PCR was carried out using the above-described cDNA as a template, and the expression level of the TRPV4 gene in the NHEK cells was quantified. The expression level of glyceraldehyde 3-phosphate dehydrogenase (GAPDH) was quantified as an internal standard gene. GAPDH is a housekeeping gene in which the expression does not change due to the addition of a compound. The expression level of the TRPV4 gene was standardized with reference to the expression level of the GAPDH gene in the NHEK cells. As a primer for amplifying the GAPDH gene, a primer GAPDH (ID: HA067812) manufactured by Takara Bio Inc. was used. As a primer for amplifying the TRPV4 gene, a primer TRPV4 (ID: HA186338) manufactured by Takara Bio Inc. was used.

The same operation as described above was carried out using, instead of δ-TPG, D-α-tocopheryl glucoside (hereinafter, may be abbreviated as "α-TPG"), tocopherol acetate (FUJIFILM Wako Pure Chemical Corporation; hereinafter, may be abbreviated as "TPA"), δ-tocopherol (Sigma-Aldrich Co., LLC; hereinafter, may be abbreviated as "δ-TP"), or α-tocopherol (SEIKO-EPSON CORPORATION; hereinafter, may be abbreviated as "α-TP"), thereby obtaining evaluation cells. The expression level of the TRPV4 gene was measured for these evaluation cells by the same operation as described above.

The expression level of the TRPV4 gene was determined as a relative value when the expression level of the TRPV4 gene in reference cells was set to 1. The reference cells were prepared by culturing the NHEK cells without adding a test compound to the cell culture solution and without irradiating the cells with UVB. HD was added to the cell culture solution of the reference cells so that the HD concentration in the cell culture solution was the same as that in the culture solution when δ-TPG was added and cultured. The culture conditions for the NHEK cells were the same as those when δ-TPG was added, except that δ-TPG was not added and UVB irradiation was not carried out.

Comparative cells were prepared by culturing the NHEK cells by not adding the test compound to the cell culture solution and irradiating the cells with UVB. HD was added to the cell culture solution of the comparative cells such that the HD concentration in the cell culture solution was the same as that in the culture solution when δ-TPG was added and cultured. The culture conditions for the comparative cells were the same as those when δ-TPG was added, except that δ-TPG was not added. The expression level of the TRPV4 gene in the comparative cells was determined by the same method as described above.

An aqueous composition containing tocopheryl glucoside and not containing HD was also prepared, but the tocopheryl glucoside was not sufficiently dissolved in the aqueous composition. Therefore, the preparation of evaluation cells and the measurement of the expression level of the TRPV4 gene were not carried out.

The results are shown in Table 3.

**[Table 3]**

| UVB irradiation | Sample | Expression level of TRPV4 gene (relative value) |
|---|---|---|
| Not performed | HD | 1.00 |
| Performed | HD | 0.34 |
| | δ-TPG/HD | 0.83 |
| | α-TPG/HD | 0.77 |
| | δ-TP/HD | 0.54 |
| | α-TP/HD | 0.43 |
| | TPA/HD | 0.29 |

The TRPV4 gene was expressed at a decreased level by irradiating with UVB. On the other hand, by adding an aqueous composition containing tocopheryl glucoside (δ-TPG or α-TPG) to the culture solution, a decrease in the expression of TRPV4 due to UVB irradiation was significantly suppressed. This result indicates that tocopheryl glucoside had an effect of significantly promoting the expression of the TRPV4 gene in the cells irradiated with UVB. The effect of promoting the expression of the TRPV4 gene by tocopheryl glucoside was significantly higher than the effect of promoting the expression of the TRPV4 gene by δ-TP, α-TP, and TPA.

### [Experimental Example 3]

(Confirmation of activity of aqueous composition with δ-TPG and HD: effect of promoting expression of TJ-related gene)
Normal human epidermal keratinocytes (NHEK, manufactured by KURABO INDUSTRIES LTD.) were subjected to the same operation as in Experimental Example 2 to obtain evaluation cells.

Total RNA was extracted from the evaluation cells thus obtained, and cDNA was synthesized. Subsequently, quantitative real-time PCR was carried out using the above-described cDNA as a template, and the expression levels of the claudin 1 (CLDN1) gene and occludin (OCL) gene in the NHEK cells were quantified. In addition, the expression level of GAPDH was quantified as an internal standard gene. The expression levels of the CLDN1 gene and OCL gene were standardized with reference to the expression level of the GAPDH gene in the NHEK cells. As a primer for amplifying the GAPDH gene, a primer GAPDH (ID: HA067812) manufactured by Takara Bio Inc. was used. As a primer for amplifying the CLDN1 gene, a primer CLDN1 (ID: HA103348) manufactured by Takara Bio Inc. was used. As a primer for amplifying the OCL gene, a primer OCL (ID: HA036753) manufactured by Takara Bio Inc. was used.

The same operation as described above was performed using, instead of δ-TPG, α-TPG, TPA, δ-TP, or α-TP to obtain evaluation cells. The expression levels of the CLDN1 gene and OCL gene were measured for these evaluation cells by the same operation as described above.

The expression level of each gene was determined as a relative value when the expression level of each gene in reference cells was set to 1. The reference cells were prepared by the same operation as the reference cells in Experimental Example 2.

The expression levels of the CLDN1 gene and OCL gene in comparative cells were determined by the same method as described above. The comparative cells were prepared by the same operation as the comparative cells in Experimental Example 2.

An aqueous composition containing tocopheryl glucoside and not containing HD was also prepared, but the tocopheryl glucoside was not sufficiently dissolved in the aqueous composition. Therefore, the preparation of the evaluation cells and the measurement of the expression levels of the CLDN1 gene and OCL gene were not carried out.

The results are shown in Table 4.

**[Table 4]**

| UVB irradiation | Sample | Expression level of gene (relative value) | |
|---|---|---|---|
| | | CLDN1 gene | OCL gene |
| Not performed | HD | 1.00 | 1.00 |
| Performed | HD | 0.58 | 0.66 |
| | δ-TPG/HD | 1.28 | 3.45 |
| | α-TPG/HD | 1.24 | 3.24 |
| | δ-TP/HD | 1.00 | 1.91 |
| | α-TP/HD | 1.00 | 2.48 |
| | TPA/HD | 0.77 | 1.12 |

By irradiating with UVB, the expression of the CLDN1 gene and OCL gene was decreased (comparison between "Without UVB irradiation and Adding sample" and "With UVB irradiation and Adding sample"). On the other hand, by adding an aqueous composition containing tocopheryl glucoside (δ-TPG or α-TPG) to the culture solution, the expression levels of the CLDN1 gene and OCL gene were significantly increased. The effect of promoting the expression of the CLDN1 gene and OCL gene by the tocopheryl glucoside was significantly higher than the effect of promoting the expression of the CLDN1 gene and OCL gene by δ-TP, α-TP, and TPA.

### [Experimental Example 4]

### (TJ-forming ability evaluation test)

A skin model (model "EP1-200", manufactured by KURABO INDUSTRIES LTD.) was pre-cultured for 1 hour under the conditions of 37°C and 5% CO₂. Subsequently, δ-TPG dissolved in a 0.5% by mass HD aqueous solution at a concentration of 0.2% by mass was added to the surface of the skin model, and the skin model was further cultured for 24 hours. Subsequently, δ-TPG was removed, the sample was washed three times with a phosphate buffer solution, and the transepidermal water loss (TEWL) was measured. A cyclone water vaporizer (manufactured by ASCH JAPAN Co.,Ltd.) was used for measuring the TEWL.

TEWL was measured by performing the same operation as described above using TPA instead of δ-TPG.

For comparison, a 0.5% by mass HD aqueous solution not containing δ-TPG instead of δ-TPG dissolved in a 0.5% by mass HD aqueous solution was used, and the same operation as described above was performed to measure the TEWL.

The same operation as described above was performed using δ-TP dissolved in 1% by mass of HD at a concentration of 0.2% by mass, instead of δ-TPG dissolved in 0.5% by mass of HD, and the TEWL was measured.

Table 5 shows the measurement results of the TEWL. It was confirmed that TEWL was suppressed in the group in which δ-TPG was added. This result indicates that the formation of TJ was promoted by δ-TPG, and thus moisturizing ability (moisture retention function) of the skin was enhanced.

**[Table 5]**

| Sample | TEWL [g/(h·m²)] |
|---|---|
| HD | 14.6 ± 1.7 |
| δ-TPG/HD | 7.9 ± 1.7 |
| δ-TP/HD | 8.2 ± 1.2 |
| TPA/HD | 12.4 ± 0.8 |

After the measurement of TEWL, each skin model was collected and paraffin-embedded, and a tissue section specimen was collected using a section preparation device.

The tissue section specimen was subjected to deparaffinization treatment and dehydration treatment to obtain a skin tissue sample. Immunohistochemical staining was carried out on the skin tissue sample. As a primary antibody, a rabbit anti-TRPV4 antibody, a rabbit anti-occludin antibody, or a rabbit anti-claudin 1 antibody (all manufactured by Abcam plc.) was used. Each antibody was diluted with a phosphate buffer solution containing 0.05% by mass Tween-20 at a dilution rate of the rabbit anti-TRPV4 antibody (1:100), the rabbit anti-occludin antibody (1:100), and the rabbit anti-claudin 1 antibody (1:100). As a secondary antibody, a peroxidase-binding donkey anti-rabbit IgG antibody (manufactured by Abcam plc.) derived from Western wasabi diluted with a phosphate buffer solution containing 0.05% by mass Tween-20 at 1:1000 was used. After the reaction of the primary antibody and the secondary antibody, the skin tissue sample was washed. Thereafter, a color development reaction was carried out using a peroxidase color developing substrate, 3,3'-diaminobenzidine tetrahydrochloride (manufactured by FUJIFILM Wako Pure Chemical Corporation).

Each skin tissue sample was observed with an inverted microscope (Nikon TS1), and photographed. For the obtained three independent images, a brightness value correlated with a protein expression level was quantitatively analyzed using Image J software (NIH).

The results are shown in Table 6. The brightness value of each gene in each example is shown as a relative value when the average brightness value of each gene in the skin tissue sample using a 0.5% by mass HD aqueous solution not containing the test compound was set to 1.00.

**[Table 6]**

| Sample | Brightness (relative value) | | |
|---|---|---|---|
| | TRPV4 | CLDN1 | OCL1 |
| HD | 1.00 | 1.00 | 1.00 |
| δ-TPG/HD | 1.25 | 1.84 | 1.44 |
| α-TPG/HD | 1.97 | 1.55 | 1.29 |
| TPA/HD | 0.11 | 0.67 | 0.46 |

In the group in which tocopheryl glucoside (δ-TPG or α-TPG) was added, it was confirmed that the protein amounts of TRPV4, CLDN1, and OCL1 increased. These results indicate that the protein amount of TRPV4 was increased by the tocopheryl glucoside, and thus the amount of moisture evaporation from the skin was suppressed. In addition, it was shown that the protein amounts of CLDN1 and OCL1 increased by tocopheryl glucoside, thereby promoting the formation of TJ, and as a result, the moisturizing ability of the skin was enhanced.

Although preferred examples of the present invention have been described above, the present invention is not limited to these examples. It is possible to add other configurations or to omit, replace, or modify the configurations described herein without departing from the spirit of the present invention. The present invention is not limited by the above description, but only by the scope of the appended claims.

## Claims

1. An aqueous composition comprising:
a glycoside of vitamin E, represented by General Formula (1);
1,2-hexanediol; and
water, 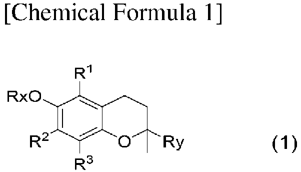
[in the formula, R¹, R², and R³ each independently represents a hydrogen atom or a methyl group, Rx represents a monosaccharide, and Ry represents a group represented by Formula (Ry-1) or (Ry-2)] 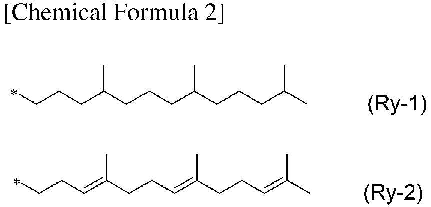
[in the formula, * represents a bonding site to a carbon atom to which Ry in General Formula (1) is bonded].

2. The aqueous composition according to Claim 1,
wherein the aqueous composition contains 0.01% to 2% by mass of the glycoside of vitamin E and 0.5% to 20% by mass of the 1,2-hexanediol.

3. The aqueous composition according to Claim 1 or 2,
wherein the glycoside of vitamin E is a glucoside of vitamin E.

4. The aqueous composition according to Claim 3,
wherein the glucoside of vitamin E is tocopheryl glucoside.

5. An external preparation for skin, comprising:
the aqueous composition according to Claim 1, 2, or 4.
